# EUROPÄISCHE PATENTANMELDUNG

(11) **EP 1 452 529 A1**
(43) Veröffentlichungstag der Anmeldung: **01.09.2004**
(21) Anmeldenummer: 04003742.6
(22) Anmeldetag: 19.02.2004
(51) Int. Cl.: C07D 277/06, C07D 263/04

(54) **Verfahren zur elektrophilen Substitution von Thiazolidinen oder Oxazolidinen**

(30) Priorität: 27.02.2003 DE 10308580
(71) Anmelder: Consortium für elektrochemische Industrie GmbH, 81379 München (DE)
(72) Erfinder: Heldmann, Dieter, Dr., 81379 München (DE); Stohrer, Jürgen, Dr., 82049 Pullach (DE)
(74) Vertreter: Schuderer, Michael, Dr.

(57) **Zusammenfassung**

Die Erfindung betrifft ein Verfahren zur Herstellung von α-funktionalisierten in 4-Position Oxycarbonyl-funktionalisierten Thiazolidinen oder Oxazolidinen durch Zugabe einer Base zu einer Reaktionsmischung enthaltend ein Oxycarbonyl-funktionalisiertes Thiazolidin oder Oxazolidin und ein Elektrophil bei einer Temperatur von größer -40°C.

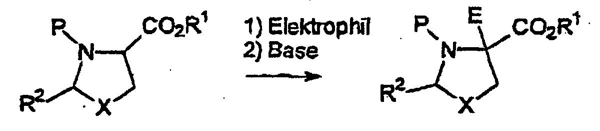

## Beschreibung

Die Erfindung betrifft ein Verfahren zur elektrophilen Substitution von Thiazolidinen oder Oxazolidinen. Insbesondere eignet sich das Verfahren zur diastereoselektiven elektrophilen Substitution von Thiazolidinen oder Oxazolidinen.

Die elektrophile α-Funktionalisierung von in 4-Position Oxycarbonyl-funktionalisierten Thiazolidinen oder Oxazolidinen ist eine bekannte Synthesestrategie zur α-Funktionalisierung von Aminosäuren. Das Verfahren eignet sich insbesondere zur Herstellung α-funktionalisierter, enantiomerenreiner, unnatürlicher Aminosäuren.

Es ist bekannt, dass man Verbindungen der allgemeinen Formel (1) beispielsweise durch Umsetzung der Ester oder der freien Säuren (R¹ kann dabei ganz allgemein für Wasserstoff, einen Silyl- oder einen organischen Rest stehen) der Aminosäuren Cystein und Serin (X steht für S oder O) im Zuge einer Kondensationsreaktion mit einem Aldeyhyd R²-CHO (R² steht für einen organischen Rest) und anschließender Einführung einer Aminoschutzgruppe P erhalten kann. Die so hergestellten Thiazolidin- oder Oxazolidin-Derivate können im folgenden durch Deprotonierung und anschließende elektrophile Substitution in 4-Position durch Einführung eines elektrophilen Restes E weiter modifiziert werden und man erhält Verbindungen der allgemeinen Formel (2).

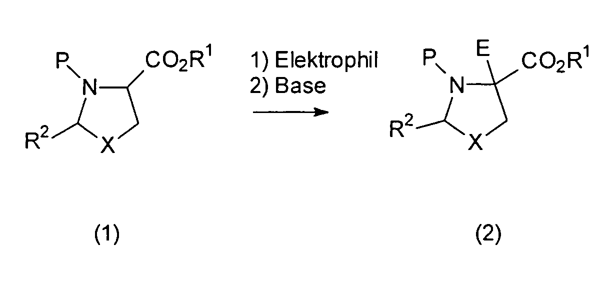

Werden zur Herstellung der Thiazolidin- oder Oxazolidin-Derivate der allgemeinen Formel (1) die natürlichen Aminosäuren L-Cystein und L-Serin bzw. deren unnatürlicher D-Formen und ein geeigneter Rest R² gewählt, so erfolgt die elektrophile Substitution an den möglichen Enantiomeren bzw. Diastereomeren resultierend aus zwei chiralen Zentren in 2- und in 4-Position des Heterocyclus diastereoselektiv in 4-Position und man erhält die Verbindungen der allgemeinen Formel (2) in Form ihrer reinen optischen Isomere. Dies sei beispielhaft in Gestalt der aus den L-Formen der Aminosäuren Cystein und Serin erhältlichen optischen Isomere der allgemeinen Formeln (1a) und (2a) veranschaulicht.

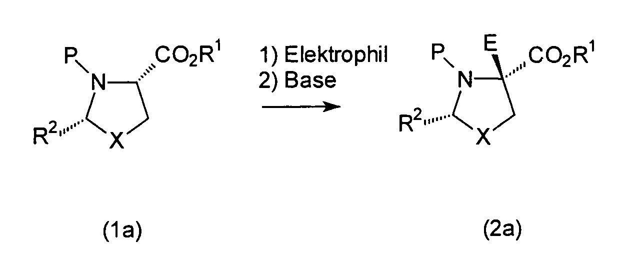

Eine abschließende hydrolytische Spaltung von Verbindungen der allgemeinen Formel (2) führt zu α-substituierten Aminosäurederivaten bzw. deren Aminhydro-Salzen der allgemeinen Formel (4).

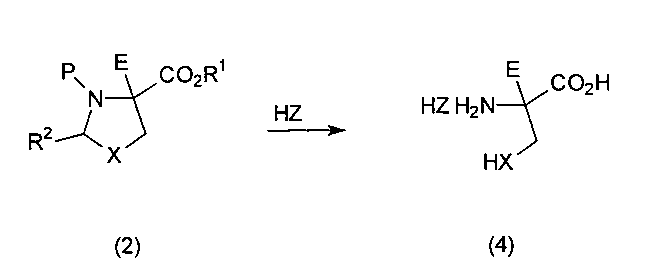

In Abhängigkeit des Rest R² werden bei zuvor diastereoselektiver Reaktionsführung auf diese Weise enantiomerenreine α-substituierte Cystein- bzw. Serinderivate (4a) erhalten wie hier beispielhaft für eine mögliche optische Konfiguration veranschaulicht.

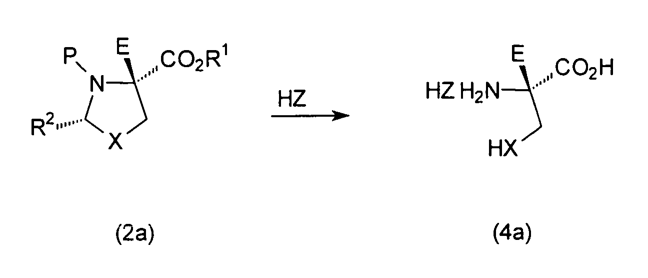

Die nach diesem allgemeinen Prinzip erhaltenen, ggf. enantiomerenreinen Aminosäurederivate sind als unnatürliche α-substituierte Aminosäuren wertvolle Zwischenprodukte für die weitere Umsetzung zu verschiedenen Pharmazeutika.

Im Stand der Technik sind eine Reihe von Verfahren zur elektrophilen Substitution in 4-Position von Verbindungen der allgemeinen Formel (1) beschrieben worden, insbesondere für den speziellen Fall der Methylierung in 4-Position des auf den Cystein-Methylester bzw. Serin-Methylester zurückzuführenden Thiazolidins (X = S) oder Oxazolidins (X = O), in dem R² für einen *tert*-Butyl-Rest und P für eine Formyl-Gruppe steht.

So beschreiben D. Seebach et al. (Tetrahedron Lett. 1984, 25, 2545 - 2548, Helv. Chim. Acta 1987, 70, 1194-1216) die Herstellung von enantiomerenreinem L-2-Methylserin durch Alkylierung des entsprechenden Oxazolidins mit Methyliodid. Hierbei wird bei -78°C eine Lösung von Lithiumdiisopropylamid in THF/Hexan mit optionalem Zusatz von Hexamethylphosphorsäuretriamid (HMPA) vorgelegt, zu dieser Lösung das Oxazolidin und nach weiteren 10 min bei -78°C das Elektrophil Methyliodid gegeben. Binnen 12h wird auf 0°C erwärmt und dann aufgearbeitet.

Die Methylierung entsprechender Thiazolidine mit Methyliodid wurde bei der Herstellung von enantiomerenreinem L-/D-2-Methylcystein-hydrochlorid von G. Pattenden et al. (Tetrahedron 1993, 49(10), 2131-2138) und G. Mulqueen et al. (Tetrahedron 1993, 49(24), 5359-5364) beschrieben. Analoge Verfahren sind auch in WO 01/72702 und WO 01/72703 beschrieben. Nach einer möglichen Variante wird das Thiazolidin in THF mit 1,3-Dimethyltetrahydro-2(1H)-pyrimidon (DMPU) als Cosolvens bei -78°C gelöst, Lithiumhexamethyldisilazid in THF zugegeben, das Elektrophil Methyliodid bei -78°C zugesetzt und abschließend nach 4h bei - 78°C auf Raumtemperatur erwärmt und aufgearbeitet.

Nach einer weiteren Variante wird LiCl in 1,2-Dimethoxyethan und THF homogen gelöst, das Thiazolidin bei -65°C in THF gelöst dazugegeben, das Elektrophil Methyliodid zugesetzt und im folgenden die Base Lithiumhexamethyldisilazid bei -65°C zugegeben und für 10h bei -65°C zur Reaktion gebracht und abschließend auf Raumtemperatur erwärmt und aufgearbeitet. Nach einer dritten Variante wird zu einer Lösung von Lithiumdiisopropylamid in Hexan/THF bei -78°C DMPU zugesetzt, auf -90°C gekühlt, dann das Thiazolidin in THF zugegeben, das Elektrophil Methyliodid bei -90°C zugesetzt und nach 2h bei -90°C auf Raumtemperatur erwärmt und aufgearbeitet.
Nach diesen Verfahren werden maximale Ausbeuten an reinem Produkt von 46-63% nach chromatographischer Aufarbeitung erhalten.

Die aus dem Stand der Technik für den Labormaßstab beschriebenen Verfahren haben insbesondere für eine großtechnische Umsetzung eine Reihe von Nachteilen. So ist die Verwendung von extrem tiefen Temperaturen im technischen Maßstab nicht realisierbar oder mit unverhältnismäßig hohen Kosten verbunden.
Die niedrigen Reaktionstemperaturen führen in der Folge zu unwirtschaftlich langen Reaktionszeiten und zu einer niedrigen Löslichkeit der Edukte in den verwendeten Lösungsmitteln, was wiederum den Einsatz großer Lösungsmittelmengen erfordert und sich schließlich negativ auf die Raum-Zeit-Ausbeute auswirkt.
Der Zusatz von reinen Hilfsstoffen, wie Cosolventien oder Lithiumsalzen, die nur als Additive eingesetzt werden und sich folglich nicht im Produkt wiederfinden, verursacht einerseits zusätzliche Kosten, andererseits müssen die Hilfsstoffe in zusätzlichen Aufarbeitungsschritten wieder vollständig vom Produkt abgetrennt werden, um die hohen Reinheitsanforderungen an pharmazeutische Zwischenprodukte zu erfüllen.

Die aus dem Stand der Technik bekannten elektrophilen Substitutionsverfahren von Verbindungen der allgemeinen Formel (1) zur Herstellung unnatürlicher, α-substituierter Aminosäuren der allgemeinen Formel (4) besitzen folglich eine Reihe von Nachteilen, die die Übertragung vom Labormaßstab in die großtechnische Umsetzung unwirtschaftlich und ineffizient machen.

Es bestand daher die Aufgabe ein großtechnisch durchführbares und wirtschaftliches Verfahren zur Herstellung von Verbindungen der allgemeinen Formel (2) bereit zu stellen.

Die Aufgabe wurde gelöst durch die Entwicklung eines Verfahrens zur elektrophilen Substitution von Verbindungen der allgemeinen Formel (1), das bei deutlich höheren Temperaturen durchgeführt werden kann und auf die Verwendung von zusätzlichen Hilfsstoffen verzichtet.

Es wurde überraschender Weise gefunden, dass durch die Wahl geeigneter Reaktionsbedingungen, insbesondere der Reaktionstemperatur, auf die aus der Literatur bekannten und einen positiven Effekt auf den Reaktionsverlauf nehmenden Hilfsstoffe, insbesondere auf Li-Salz-Zusätze, die zur Bildung intermediärer Lithium-Enolate führen, wie von D. Seebach et al. (Angew. Chem. 1988, 100, 1685-1715) beschrieben wurde, verzichtet werden kann.

Gegenstand der Erfindung ist ein Verfahren zur Herstellung von Verbindungen der allgemeinen Formel (2) 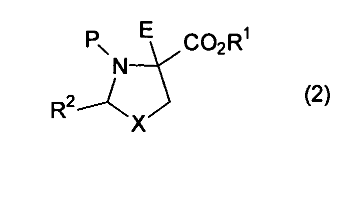 wobei
- **X**: für S oder O steht und
- **R**^{**1**}: ausgewählt wird aus der Gruppe enthaltend Wasserstoff, Metalle der ersten oder zweiten Hauptgruppe, lineare oder verzweigte C₁-C₁₂-Alkyl-, C₆-C₁₅-Aryl- oder C₇-C₂₁ Aralkyl-Reste, Dialkylsilyl-, Trialkylsilyl-, Dialkylarylsilyl-, Diarylalkylsilyl-, Triarylsilyl-Reste, wobei die organischen Reste an den Silylresten wiederum aus C₁-C₁₂-Alkyl und C₆-C₁₅-Aryl-Resten ausgewählt werden und
- **R**^{**2**}: ausgewählt wird aus der Gruppe enthaltend lineare oder verzweigte C₁-C₁₂-Alkyl-, C₆-C₁₅-Aryl- oder C₇-C₂₁ Aralkyl-Reste und
- **P**: für eine Aminoschutzgruppe steht und
- **E**: für einen Rest ausgewählt aus der Gruppe enthaltend gegebenenfalls durch Halogen-, Cyano-, Nitro- oder Ester-Gruppen substituierte, lineare oder verzweigte C₁-C₁₂-Alkyl-, C₃-C₁₀-Alkenyl-, C₆-C₁₅-Aryl- oder C₇-C₂₁-Aralkyl-Reste oder für eine Acyl- oder Formylgruppe steht
durch Zugabe einer Base zu einer Reaktionsmischung enthaltend eine Verbindung der allgemeinen Formel (1) 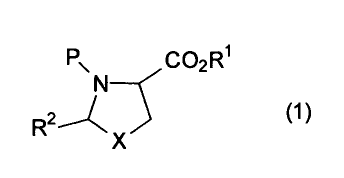 und ein Elektrophil E-Y
wobei
wobei
**Y** für eine Abgangsgruppe steht,
dadurch gekennzeichnet, dass die Reaktion bei einer Temperatur von größer - 40 °C durchgeführt wird.

In einer möglichen Ausführungsform des erfindungsgemäßen Verfahrens wird dieses diastereoselektiv durchgeführt.

Das erfindungsgemäße Verfahren kann dabei in analoger Weise auf alle anderen Diastereomere oder Enantiomere der Edukte der allgemeinen Formel (1) mit den Formel (1a), (1b), (1c) oder (1d) angewendet werden, sofern diese in optisch reiner Form vorliegen oder als Gemisch, welches zumindest eine einheitliche Konfiguration an C-2 besitzt; entweder nur (2R)- oder nur (25), da dieses stereogene Zentrum die optische Induktion der Substitution des Elektrophils bestimmt.

So führen die Edukte mit den allgemeinen Formeln (1a) und (1b) oder deren Gemische zu Verbindungen der allgemeinen Formel (2a) und Edukte mit den allgemeinen Formeln (1c) und (1d) oder deren Gemische zu Verbindungen der allgemeinen Formel (2b).

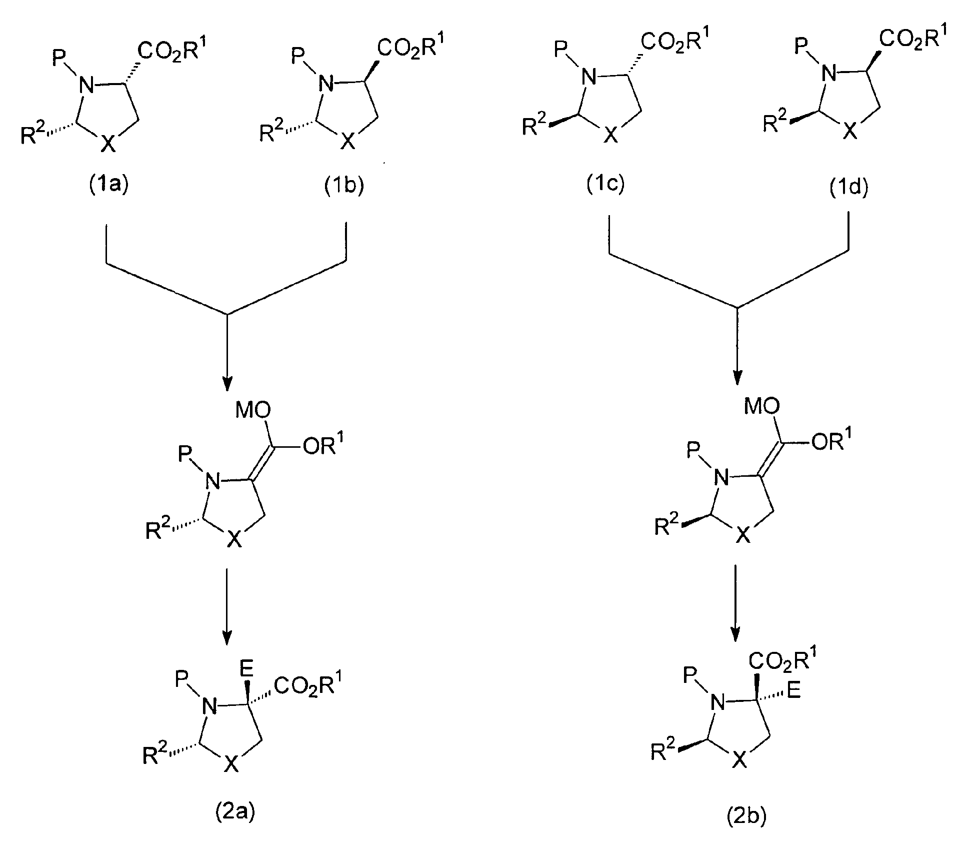

Generell wird nach dem erfindungsgemäßen Verfahren das umzusetzende Edukt der allgemeinen Formel (1) oder dessen optisch reine Formen oder dessen entsprechende Gemische zusammen mit dem Elektrophil vorteilhafterweise gelöst in einem Lösungsmittel vorgelegt und anschließend die Base zugegeben.

Der Zusatz der Base führt zur Bildung eines planaren, enolatstabilisierten Anions in 4-Position des Rings und in α-Stellung zur Oxycarbonyl-Funktionalität, welches in-situ durch das Elektrophil unter Bildung einer Verbindung der allgemeinen Formel (2) abgefangen wird, wobei die Diastereoselektivität der Reaktion durch das intakte enantiomerenreine stereogene Zentrum in 2-Position des Ringes bewirkt wird.

Das erfindungsgemäße Verfahren bietet durch einfache Hydrolyse der erhaltenen Verbindungen der allgemeinen Formel (2) bzw. der optischen Isomere (2a) oder (2b) einen effizienten, großtechnisch einfach realisierbaren, hoch diastereoselektiven und letztlich wirtschaftlichen Zugang zu unnatürlichen, α-substituierten Aminosäuren der allgemeinen Formel (4), insbesondere deren enantiomerenreinen Formen wie beispielsweise dem L-2-Methylserin Hydrochlorid oder L-2-Methylcystein Hydrochlorid.

Das erfindungsgemäße Verfahren kann bei deutlich höheren Temperaturen und ohne den Zusatz von Hilfsstoffen bei gleichzeitig hoher Diastereoselektivität durchgeführt werden und umgeht so die aus dem Stand der Technik bekannten Nachteile, die die Umsetzung in ein großtechnisches Verfahren verhinderten.

In einer bevorzugten Ausführungsform des erfindungsgemäßen Verfahrens werden optische Isomere in der Konfiguration der allgemeinen Formel (2a) erhalten 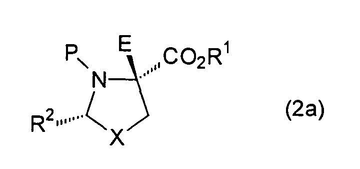 wobei als Edukte optische Isomere der allgemeinen Formeln (1a) oder (1b) in reiner Form oder als Gemisch 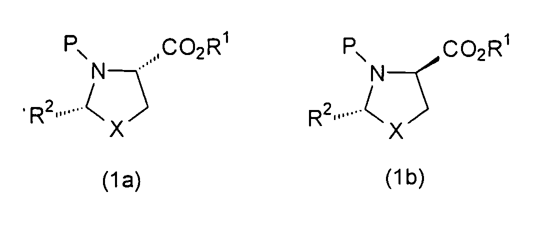 eingesetzt werden und die Reste R¹, R², P, E und X die oben genannten Bedeutungen haben, insbesondere aus den unten aufgeführten jeweils besonders bevorzugten Ausführungsformen ausgewählt werden.

In einer besonders bevorzugten Ausführungsform des erfindungsgemäßen Verfahrens können durch Verwendung von Pivalaldehyd (R² = *tert*-Butyl), den Methylestern von L-Serin oder L-Cystein, einer Formyl-Schutzgruppe und Methyliodid als Elektrophil mit Lithiumhexamethyldisilazid (LiHMN) als Base und abschließender saurer Hydrolyse mit Salzsäure L-2-Methylcystein Hydrochlorid bzw. L-2-Methylserin Hydrochlorid gemäß dem folgenden Reaktionsschema hergestellt werden:

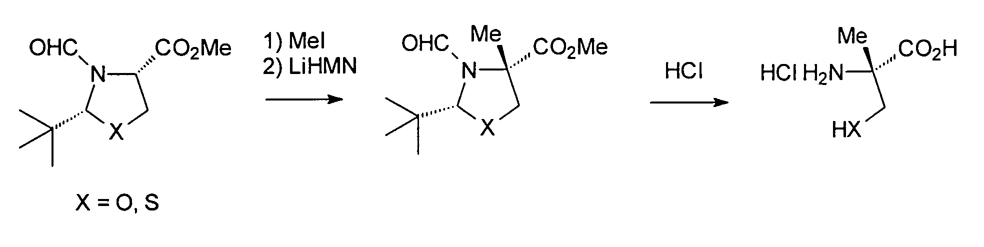

L-2-Methylcystein bzw. L-2-Methylserin, die durch vollständige Hydrolyse aus dem methylierten Thiazolidin bzw. Oxazolidin (E = Me) erzeugt werden können, können direkt in weiteren Umsetzungen zur Erzeugung von Pharmazeutika eingesetzt werden.

Das für das erfindungsgemäße Verfahren einzusetzende Elektrophil E-Y wird dabei generell so ausgewählt, dass es in der Lage ist, Metallenolate zu substituieren.

Der durch das Elektrophil eingeführte Rest E steht dabei bevorzugt für gegebenenfalls durch Halogen-, Cyano-, Nitrooder Ester-Gruppen funktionalisierte, lineare oder verzweigte C₁-C₁₂-Alkyl-, insbesondere Methyl-, Ethyl-, Propyl-, Butyl-, Pentyl-, Hexyl-, Heptyl-, Octyl-, Nonyl- oder Isopropyl-, C₆-C₁₅-Aryl-, C₇-C₂₁-Aralkyl-, insbesondere Benzyl- oder C₃-C₁₀-Alkenyl-, insbesondere Allyl-Reste oder eine Acyl- oder Formylgruppe.

Die Abgangsgruppe Y wird bevorzugt ausgewählt aus der Gruppe enthaltend Halogene, Tosylate, Stickstoffverbindungen, insbesondere Azide, Hydrazide, Dialkylamide oder Sulfonate, insbesondere Chlorid, Bromid, Iodid oder Alkylsulfonat, ganz besonders bevorzugt Iodid.

Durch eine sich anschließende wässerige Hydrolyse werden dann die eigentlichen Reaktionsprodukte freigesetzt.

Die folgenden Reste E können nach dem erfindungsgemäßen Verfahren besonders vorteilhaft eingeführt werden und sind in keiner Weise als Einschränkung zu verstehen:

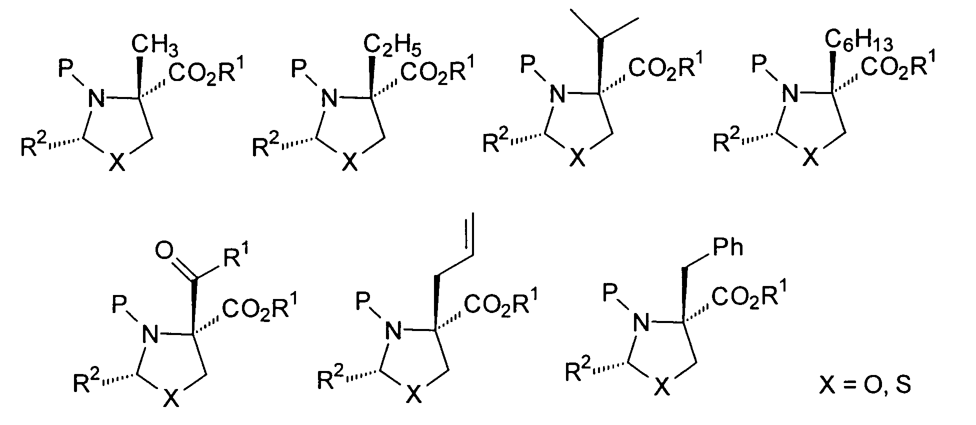

Besonders bevorzugte Elektrophile E-Y sind Methyliodid, Methylbromid, Methylchlorid, Methyltosylat, Methylnonaflat, Dimethylsulfat, insbesondere Dimethylsulfat und Methyliodid, Benzyliodid, Benzylbromid, Benzylchlorid, Tolylbromid, Tolylchlorid, 2-Bromessigsäureethylester, 2-Iodessigsäureethylester, Ethyliodid, Ethylbromid, Ethyltriflat, Propyliodid, Propylbromid, Isopropyliodid, Isopropyltriflat, Hexyliodid, Hexyltriflat, Allylchlorid, Allylbromid oder Allyliodid, Allyltriflat, Dimethylformamid und Säurechloride wie Acetylchlorid, Propansäurechlorid, Butansäurechlorid, Hexansäurechlorid, Octansäurechlorid, Pivalinsäurechlorid, Benzoylchlorid, 4-Methylbenzoesäurechlorid.

Zum Schutz der Aminofunktion gegen die eingesetzte Base wird diese in den Verbindungen der allgemeinen Formel (1) und (2) oder deren reinen optischen Isomeren mit einer Aminoschutzgruppe P geschützt. Aminoschutzgruppe ist nicht darauf beschränkt, kann aber jede Schutzgruppe sein, die gewöhnlicherweise zur Schützung von Aminogruppen verwendet wird. Für das erfindungsgemäße Verfahren können alle gängigen Aminoschutzgruppen eingesetzt werden, die dem Fachmann aus Protecting Groups, P. J. Kocienski, Thieme Verlag, 1994, S. 185-243 bekannt sind.

In einer bevorzugten Ausführungsform des erfindungsgemäßen Verfahrens werden durch N-Acyl-, N-Sulfonyl-, N-Sulfenyl, N-Silylderivate oder N-Alkyl-Gruppen geschützte Verbindungen der allgemeinen Formel (1) eingesetzt.

Besonders bevorzugte Aminoschutzgruppen P sind Formyl, Acetyl, Trifluoracetyl, Methoxycarbonyl, Ethoxycarbonyl, tert-Butoxycarbonyl, Benzyloxycarbonyl, Allyloxycarbonyl, Benzyl, Trityl, Trialkylsilyl- wie Trimethylsilyl, Triethylsilyl, Triisopropylsilyl, tert.-Butyldimethylsilyl, Aryldialkylsilylwie Phenyldimethylsilyl, Diarylalkylsilyl- wie Diphenylmethylsilyl, Triarylsilyl- wie Triphenylsilyl, insbesondere Formyl und Acetyl.

Die Reste für R¹ können aus einer Vielzahl von Möglichkeiten ausgewählt werden, so dass sich eine Vielzahl von Stoffklassen ergibt. Möglich sind organische oder Silylester, letztere insbesondere bei der Wahl anfänglich nicht saurer Reaktionsbedingungen, freie Säuren, die bei der Umsetzung mit der Base dann Dianionen bilden oder Mono- oder Dicarboxylate der freien Säure mit Metallen der ersten oder zweiten Hauptgruppe.

Bevorzugte Reste für R¹, die in dem erfindungsgemäßen Verfahren eingesetzt werden können, sind Wasserstoff, Lithium, Natrium, Kalium, Magnesium und Calcium.

Weitere bevorzugte Reste für R¹ sind aus der Klasse der organischen Reste Methyl, Ethyl, n-Propyl, Isopropyl, n-Butyl, tert-Butyl, Phenyl oder Benzyl und aus der Klasse der Silylreste Trimethylsilyl, Triethylsilyl, Tributylsilyl, Dimethylsilyl, Diphenyl, Tert.-butyldimethylsilyl, Thexyldimethylsilyl, Norbornyldimethylsilyl, Dimethylphenylsilyl, Diphenylmethylsilyl, Triphenylsilyl. Im Falle von Diorganosilyl-Resten, wie beispielsweise Dimethylsilyl- und Diphenylsilyl, resultieren Verbindungen der folgenden Struktur, die sich in analoger Weise nach dem erfindungsgemäßen Verfahren umsetzen lassen und somit als Edukte im Sinne der Verbindungen der allgemeinen Formel (2), insbesondere in einer optisch reinen Konfiguration einsetzen lassen.

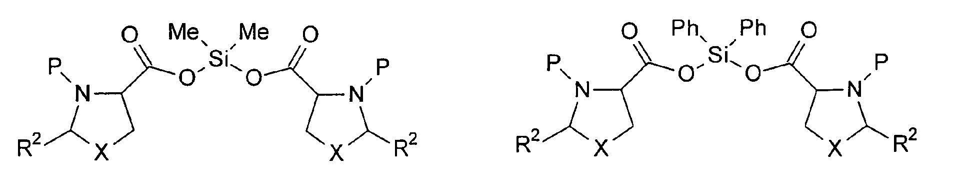

Besonders bevorzugte Reste für R¹ sind Methyl oder Ethyl.

Bevorzugte Reste für R², die in dem erfindungsgemäßen Verfahren eingesetzt werden können sind Methyl, Ethyl, n-Propyl, Isopropyl, n-Butyl, tert-Butyl, Cyclohexyl, Phenyl, Tolyl, Naphthyl oder Benzyl. Besonders bevorzugt ist tert-Butyl.

Für das erfindungsgemäße Verfahren können alle dem Fachmann bekannten Basen, die zur Erzeugung eines Metallenolates geeignet sind, eingesetzt werden.

In einer bevorzugten Ausführungsform des erfindungsgemäßen Verfahrens werden Alkali- oder Erdalkalimetallbasen, besonders bevorzugt Lithium-, Natrium- und Kaliumverbindungen, insbesondere n-, sec- oder tert-Butyllithium, Kalium-tertbutoxid, Natriumhydrid oder tert-Butylmagnesiumchlorid eingesetzt.

In einer besonders bevorzugten Ausführungsform des erfindungsgemäßen Verfahrens werden nicht-nukleophile Basen der allgemeinen Formel (3) 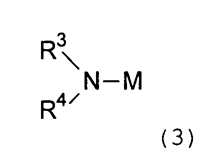 wobei R³ und R⁴ unabhängig voneinander aus der Gruppe enthaltend Alkyl, Aralkyl, Aryl oder Silyl ausgewählt werden oder R³ und R⁴ zusammen auch einen Cycloalkylrest bilden können, in dem gegebenenfalls die CH₂-Gruppen des Rings durch SiMe₂-Gruppen oder Sauerstoff subsituiert sein können und M ausgewählt wird aus der Gruppe enthaltend Lithium, Natrium, Kalium oder MgY,
wobei Y wiederum ausgewählt werden kann aus der Gruppe enthaltend Chlorid, Bromid, Iodid, oder einem zweiten Amid-Rest NR³R⁴.

Besonders bevorzugt stehen R³ und R⁴ für Trimethylsilyl oder Isopropyl. Besonders bevorzugt steht M für Lithium, Natrium oder Kalium.

Insbesondere eignen sich aus der Gruppe der Alkalimetallamide und Alkalimetallsilazide, Lithium-, Natrium- oder Kaliumdiisopropylamid, Lithium-, Natrium- oder Kaliumhexamethyldisilazid, Lithium-, Natrium- oder Kaliumcyclohexylamid und aus der Gruppe der Erdalkalimetallamide und Erdalkalimetallsilazide, Chlormagnesiumdiisopropylamid, Brommagnesiumdiisopropylamid, Magnesiumdiisopropylamid, Chlormagnesiumdicyclohexylamid, Chlormagnesiumtert-butylamid, Chlormagnesiumhexamethyldisilazid.

Ferner eignen sich für das erfindungsgemäße Verfahren besonders Alkoxyverbindungen, insbesondere Magnesiummethoxid, Magnesiumethoxid, Kaliumethoxid, Kaliummethoxid, Natriumethoxid oder Natriummethoxid.

Für das erfindungsgemäße Verfahren können die vorgenannten Basen einzeln oder in Form von Mischungen eingesetzt werden.

Als Lösungsmittel können für das erfindungsgemäße Verfahren alle unter den Reaktionsbedingungen inerten Lösungsmittel eingesetzt werden.

Besonders eignen sich Lösungsmittel aus der Klasse der Ether und Polyether, insbesondere Methyl-tert-butylether, Diethylether, Dipropylether, Dibutylether, Tetrahydrofuran, Dioxan oder 1,2-Dimethoxyethan.

Die vorgenannten Lösungsmittel können dabei alleine oder als Mischung mit anderen Lösungsmitteln ausgewählt aus der Gruppe enthaltend aromatische oder aliphatische Kohlenwasserstoffe, die als Lösungsmittel für die Base verwendet werden. Insbesondere können dabei C₁-C₁₂-Alkane, besonders bevorzugt Pentan, Hexan, Heptan, Octan, Nonan oder deren verzweigte Isomere eingesetzt werden.

Ganz besonders bevorzugt eignen sich für das erfindungsgemäße Verfahren Tetrahydrofuran oder Mischungen von Tetrahydrofuran mit Hexan.

In einer möglichen Ausführungsform des erfindungsgemäßen Verfahrens wird das Edukt der allgemeinen Formel (1) oder dessen optisch reinen Isomere in einem geeigneten Lösungsmittel gelöst und mit dem Elektrophil versetzt.

Vom Elektrophil werden bezogen auf das Edukt der allgemeinen Formel (1) oder dessen optisch reinen Isomere 1 bis 10 Äquivalente, bevorzugt 1 bis 2 Äquivalente eingesetzt.

Die Temperatur liegt sowohl bei der Zugabe des Elektrophils wie auch im folgenden bei der Zugabe der Base zwischen -40°C und +100°C, bevorzugt zwischen -30°C und +30°.

Durch die gegenüber dem Stand der Technik deutlich höhere Reaktionstemperatur des erfindungsgemäßen Verfahrens kann die Konzentration der Reaktanden deutlich höher gewählt werden. Sie liegt in einer typischen Ausführungsform des erfindungsgemäßen Verfahrens zwischen 0.1 und 5 mol/l, bevorzugt zwischen 0.2 und 1 mol/l.

Durch Zugabe der Base zu der vorgelegten Mischung der Verbindung der allgemeinen Formel (1) oder dessen optisch reinen Isomeren und dem Elektrophil wird intermediär ein Metallenolat gebildet, welches in-situ durch das Elektrophil abgefangen wird.

Im erfindungsgemäßen Verfahren beträgt die Menge an zugesetzter Base 1 bis 5 Äquivalente bezogen auf die Verbindung der allgemeinen Formel (1) oder deren optisch reine Isomere, bevorzugt 1 bis 2 Äquivalente.

Die Basen können als Feststoff oder gelöst in einem Lösungsmittel eingesetzt werden. Bevorzugt ist die Verwendung von Lösungen der Basen in inerten Lösungsmitteln, insbesondere Ethern, Polyethern, Alkanen oder Aromaten.

In Abhängigkeit der gewählten Temperatur wird die Reaktionsmischung zwischen 0 min und 4 h nachgerührt, bevorzugt sind Nachreaktionszeiten von weniger als 2h. Eine Verlängerung der Reaktionszeit ist ohne negativen Einfluss auf die Ausbeute am gewünschten Produkt.

Die Aufarbeitung und Isolierung der Verbindungen der allgemeinen Formel (2) oder deren optisch reinen Isomere erfolgt durch einfache Hydrolyse. Im allgemeinen wird die Reaktionsmischung dabei durch Zusatz eines protischen Lösungsmittels, insbesondere Wasser oder eines Alkohols hydrolisiert. In einer möglichen Ausführungsform können auch wässerige Lösungen von Basen, insbesondere NH₃, NaOH, KOH oder Säuren, insbesondere HCl, H₂SO₄, HOAc eingesetzt werden.
Die organische Phase wird anschließend salzfrei gewaschen, gegebenenfalls mit weiterem organischem Lösungsmittel verdünnt und die Phasen getrennt. Die organische Phase wird durch azeotrope Destillation oder mit einem Trockenmittel getrocknet.

Das Entfernen des Lösungsmittels führt zu den Verbindungen der allgemeinen Formel (2) oder deren optisch reinen Isomeren, welche entweder direkt weiter umgesetzt werden oder gegebenenfalls durch geeignete Reinigungsoperationen wie Destillation und Umkristallisation weiter gereinigt werden können.

Die erhaltenen Verbindungen der Formel (2) oder deren optisch reinen Isomere, wie z.B (2a), können nach literaturbekannten Verfahren, insbesondere durch salzsaure Hydrolyse zu α-substituierten Aminosäuren der allgemeinen Formel (4) oder deren optisch reinen Isomeren, wie z. B. (4a), umgesetzt werden.

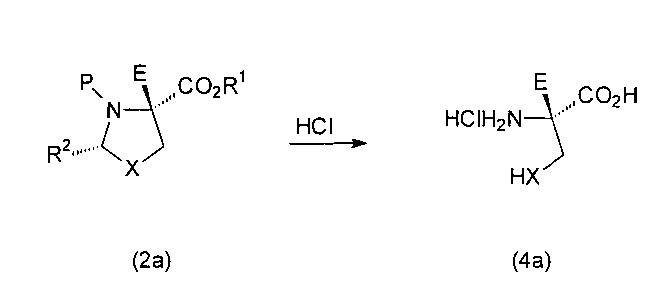

Bekannte Verfahren zur Herstellung von Verbindungen der allgemeinen Formel (4) oder deren optisch reinen Isomeren aus Verbindungen der allgemeinen Formel (2) oder deren optisch reinen Isomeren sind dem Fachmann von G. Pattenden et al. (Tetrahedron 1993, 49(10), 2131-2138), G. Mulqueen et al. (Tetrahedron 1993, 49(24), 5359-5364) oder aus WO 01/72702 bekannt.

Im allgemeinen werden dabei die Verbindungen der allgemeinen Formel (2) oder deren optisch reinen Isomeren in ca. 0.3 mol/l in 5M HCl gelöst und unter Rückfluß erhitzt. Dabei wird der Heterocyclus gespalten und in der Regel die Schutzgruppe sowie die Esterfunktion abgespalten. Wenn säurestabile Schutzgruppen P und/oder R¹ verwendet werden, muss in einem zusätzlichen Schritt zur Abspaltung dieser Schutzgruppen nach literaturbekannter, geeigneter Weise wie insbesondere für Aminoschutzgruppen P in Protecting Groups, P. J. Kocienski, Thieme Verlag, 1994, 185-243 und Esterfunktionen R¹ in Protecting Groups, P. J. Kocienski, Thieme Verlag, 1994, 118-154 beschrieben, vorgegangen werden.

Die folgenden Beispiele dienen der detaillierten Erläuterung der Erfindung und sind in keiner Weise als Einschränkung zu verstehen.

### Beispiele

### Beispiel 1

***(2R,4R)-2-Tert.-butyl-3-formyl-1,3-thiazolidin-4-allyl-4-carbonsäuremethylester:*** In einem Laborreaktor wurde (2R,4R)-2-Tert.-butyl-3-formyl-1,3-thiazolidin-4-carbonsäuremethylester (20,8 g) vorgelegt, in wasserfreiem THF (100 ml) gelöst und auf -25°C abgekühlt. Allylbromid (8,5 ml) wurde zudosiert. Anschließend wurde Lithiumhexamethyldisilazidlösung (100 ml, 1M in THF) langsam zudosiert. Die Reaktionsmischung wurde mit 15%iger Essigsäure gequencht, die Phasen getrennt und die organische Phase eingedampft, in MTBE aufgenommen, mit H₂O gewaschen und wieder eingedampft. Ausbeute 23,2 g bräunliches Öl (95%) (2R,4R)-2-Tert.-butyl-3-formyl-1,3-thiazolidin-4-allyl-4-carbonsäuremethylester.
HPLC-Gehalt Rohprodukt: Edukt < 0.5%, Produkt 92.6%, zwei nicht identifizierte Peaks mit 1.5 und 4.5%.
NMR (CDCl₃, 300 MHz): Konformerenverhältnis 53:47; 0.94 und 1.06 (9 H, s, C(CH₃)₃), 2.80 - 3.12 (3 H, m, CH₂CHCH₂ und CH₂S), 3.28 und 3.70 (1 H, d, CH₂S), 3.78 und 3.82 (3 H, s, CO₂CH₃), 4.65 und 5.37 (1 H, s, CHC(CH₃)₃), 5.05 - 5.25 (2 H, m, CH₂CHCH₂), 5.70 - 5.85 und 5.95 - 6.12 (2 H, m, CH₂CHCH₂), 8.35 und 8.52 (1 H, s, CHO).

### Beispiel 2

***(2R,4R)-2-Tert.-butyl-3-formyl-1,3-thiazolidin-4-benzyl-4-carbonsäuremethylester:*** In einem Laborreaktor wurde (2R,4R)-2-Tert.-butyl-3-formyl-1,3-thiazolidin-4-carbonsäuremethylester (40 g) vorgelegt, in wasserfreiem THF (200 ml) gelöst und auf -25°C abgekühlt. Benzylbromid (22,7 ml) wurde zugegeben. Die Lithiumhexamethyldisilazidlösung (190 ml, 1M in THF) wurde langsam zudosiert. Nach Reaktionsende wurde die Reaktionsmischung mit 15%iger Essigsäure gequencht, die Phasen getrennt und die organische Phase eingedampft, in MTBE aufgenommen, mit H₂O gewaschen und wieder eingedampft. Das erhaltene Öl kristallisierte langsam durch und wurde mit Petrolether verrieben. Ausbeute 42,0 g oranger Feststoff (77%) (2R,4R)-2-Tert.-butyl-3-formyl-1,3-thiazolidin-4-benzyl-4-carbonsäuremethylester.
HPLC-Gehalt Rohprodukt: Produkt 96.2%, Edukt nicht nachweisbar, Benzylbromid 0.8%.Nach Umkristallisation aus Petrolether/Ethylacetat: farblose Kristalle, Reinheit > 99.5%. NMR (CDCl₃, 300 MHz): Konformerenverhältnis 60:40; 0.94 und 1.03 (9 H, s, C(CH₃)₃), 2.95 -3.65 (4 H, m, 2 x CH₂), 3.76 und 3.78 (3 H, s, CO₂CH₃), 4.50 und 5.36 (1 H, s, CHC(CH₃)₃), 7.05 - 7.14 (1 H, m, ArH), 7.20 -7.36 (4 H, m, ArH), 8.45 und 8.67 (1 H, s, CHO).

### Beispiel 3

***(2R,4R)-2-Tert.-butyl-3-formyl-1,3-thiazolidin-4-benzoyl-4-carbonsäuremethylester:*** Im Laborreaktor wurde (2R,4R)-2-Tert.butyl-3-formyl-1,3-thiazolidin-4-carbonsäuremethylester (20,8 g) vorgelegt, in wasserfreiem THF (100 ml) gelöst auf -25°C abgekühlt. Benzoylchlorid (11,6 ml) wurde zudosiert. Die Lithiumhexamethyldisilazidlösung (100 ml, 1M in THF) wurde dann langsam zudosiert. Die Reaktionsmischung wurde mit 15%iger Essigsäure gequencht, die Phasen getrennt und die organische Phase eingedampft, erneut in MTBE aufgenommen, mit H₂O gewaschen und wieder eingedampft. Ausbeute 34,5 g braunes Öl (quantitativ, enthielt noch Essigsäurereste) (2R,4R)-2-Tert.butyl-3-formyl-1,3-thiazolidin-4-benzoyl-4-carbonsäuremethylester.
HPLC-Gehalt Rohprodukt: Edukt nicht nachweisbar, Produkt 82,8%, Rest mehrere nicht identifizierte kleinere Peaks.
Nach chromatographischer Reinigung: 97.8%.
NMR (CDCl₃, 300 MHz): Konformerenverhältnis 79:21; 1.00 und 1.06 (9 H, s, C(CH₃)₃), 3.41 (1 H, d, CH₂), 4.36 (1 H, d, CH₂), 3.80 und 3.83 (3 H, s, CO₂CH₃), 4.95 und 5.54 (1 H, s, CHC(CH₃)₃), 7.35 - 7.65 (3 H, m, ArH), 7.90 - 8.05 (4 H, m, ArH), 8.30 und 8.43 (1 H, s, CHO).

### Beispiel 4

***(2R,4R)-2-Tert.-butyl-3-formyl-1,3-thiazolidin-4-ethyl-4*-*carbonsäuremethylester:*** In einem Laborreaktor wurde (2R,4R)-2-Tert.-butyl-3-formyl-1,3-thiazolidin-4-carbonsäuremethylester (37,5 g) vorgelegt, in wasserfreiem THF (180 ml) gelöst und auf -25°C abgekühlt. Trifluormethansulfonsäureethylester (23 ml) wurde zudosiert. Die Lithiumhexamethyldisilazidlösung (180 ml, 1M in THF) wurde dann langsam zudosiert. Die Reaktionsmischung wurde mit 15%iger Essigsäure gequencht, mit ges. NaCl-Lsg. verdünnt, die Phasen getrennt und die organische Phase eingedampft, in MTBE aufgenommen, mit H₂O gewaschen und im Vakuum aufkonzentriert. Ausbeute 43,5 g oranges Öl (quantitativ, enthielt noch Essigsäure) (2R,4R)-2-Tert.-butyl-3-formyl-1,3-thiazolidin-4-ethyl-4-carbonsäuremethylester.
HPLC-Gehalt Rohprodukt: 2.1% Edukt, 94.3% Produkt, zwei weitere Peaks (1.2%, 2.2%) nicht identifizierbar.
NMR (CDCl₃, 300 MHz): Konformerenverhältnis 76:24; 0.95 und 1.05 (9 H, s, C(CH₃)₃), 1.01 (3 H, t, CH₂CH₃), 2.12 (2 H, m, CH₂CH₃), 3.00 und 3.35 (1 H, d, CH₂), 3.73 (1H, d, CH₂), 3.78 und 3.81 (3 H, s, CO₂CH₃), 4.72 und 5.42 (1 H, s, CHC(CH₃)₃), 8.40 und 8.47 (1 H, s, CHO).

### Beispiel 5

***(2R,4R)-2-Tert.-butyl-3-formyl-1,3-thiazolidin-4-methyl-4-carbonsäuremethylester:*** n-BuLi (1.6M in Hexan, 100 ml) wurde vorgelegt und unter Eiskühlung 1,1,3,3-Hexamethyldisilazan (35,5 ml) zugetropft. Die Lösung wurde auf RT erwärmt. In einem zweiten Kolben wurde (2R,4R)-2-Tert.-butyl-3-formyl-1,3-thiazolidin-4-carbonsäuremethylester (31,9 g) vorgelegt, in wasserfreiem THF (190 ml) gelöst und auf -28°C abgekühlt. Methyliodid (10,0 ml) wurde zudosiert. Die Lithiumhexamethyldisilazidlösung wurde dann zudosiert und der Reaktionsverlauf per GC verfolgt. Nach kompletter Umsetzung wurde die Reaktionsmischung wurde mit verdünnter Essigsäure gequencht, die Phasen getrennt und die organische Phase eingedampft, in MTBE aufgenommen und mit Wasser gewaschen und im Vakuum aufkonzentriert. Ausbeute 31,1 g (92%) (2R,4R)-2-Tert.-butyl-3-formyl-1,3-thiazolidin-4-methyl-4-carbonsäuremethylester. GC-Gehalt Rohprodukt: 0.3% Edukt, 97.1% Produkt, 1,7% N-Formyl-N-(2-methylthio-3,3-dimethyl-1-propyl)-dehydroalaninmethylester.
NMR (CDCl₃, 300 MHz): Konformerenverhältnis 70:30; 0.96 und 1.07 (9 H, s, C(CH₃)₃), 1.67 und 1.70 (3 H, s, CH₃), 2.72 und 2.86 (1 H, m, CH₂), 3.32 und 3.65 (1 H, d, CH₂), 3.76 und 3.83 (3 H, s, CO₂CH₃), 4.66 und 5.30 (1 H, s, CHC(CH₃)₃), 8.29 und 8.42 (1 H, s, CHO).

### Beispiel 6

***L-2-Methylcystein Hydrochlorid:*** Ein Teil des Produktes (15 g) wurde mit 5M HCl (175 ml) übergossen und 3d am Rückfluss gekocht. Danach wurde die Salzsäure abdestilliert und das Produkt zur Trockne eingedampft und durch Kratzen zur Kristallisation gebracht. L-2-Methylcystein, beiges Pulver (9,7 g, 92%). Ein Teil wurde zur ee-Bestimmung mit Formaldehyd derivatisiert und per HPLC analysiert (Chirobiotic T, 250 x 4.6 mm, Astec): 99,3%ee.

### Beispiel 7

***(2R,4S)-2-Tert.-butyl-3-formyl-1,3-oxazolidin-4-methyl-4-carbonsäuremethylester:*** In einem Laborreaktor wurde (2R,4S)-2-Tert.-butyl-3-formyl-1,3-oxazolidin-4-carbonsäuremethylester (19,4 g) vorgelegt, in wasserfreiem THF (100 ml) gelöst und auf -25°C abgekühlt. Methyliodid (6,2 ml) wurde zudosiert. Die Lithiumhexamethyldisilazidlösung (100 ml, 1M in THF) wurde dann langsam zudosiert. Nach Aufwärmen wurde die Reaktionsmischung mit 15%iger Essigsäure gequencht, mit ges. NaCl-Lsg. verdünnt, die Phasen getrennt und die organische Phase eingedampft, in MTBE aufgenommen, mit H₂O gewaschen. Die vereinigten organischen Phasen wurden einmal mit MTBE reextrahiert und alle organischen Phasen im Vakuum aufkonzentriert. Ausbeute 16,1 g (78%) (2R,4S)-2-Tert.-butyl-3-formyl-1,3-oxazolidin-4-methyl-4-carbonsäuremethylester. GC-Gehalt Rohprodukt: 1.8%, 5.3%, 1.7% (drei nicht identifizierte Nebenprodukte), 82.7% Produkt, 3.8% nicht identifiziertes Nebenprodukt.
Aus der Menge an isoliertem Rohprodukt (78%) und dessen Gehalt (82.7%) ergibt sich eine theoretische Ausbeute von 65% Reinprodukt.
NMR (CDCl₃, 300 MHz): Konformerenverhältnis 54:46; 0.92 und 1.02 (9 H, s, C(CH₃)₃), 1.68 und 1.69 (3 H, s, CH₃), 3. 60 - 3.80 (1 H, m, CH₂), 4.30 und 4.68 (1 H, d, CH₂), 3.75 und 3.78 (3 H, s, CO₂CH₃), 4.93 und 5.28 (1 H, s, CHC (CH₃)₃), 8.37 und 8.49 (1 H, s, CHO).

### Beispiel 8

***(2R,4R)-2-Tert.-butyl-3-formyl-1,3-thiazolidin-4-methyl-4-carbonsäuremethylester:*** In einem Laborreaktor wurde (2R,4R)-2-Tert.-butyl-3-formyl-1,3-thiazolidin-4-carbonsäuremethylester (20,8 g) vorgelegt, in wasserfreiem THF (130 ml) gelöst und auf -25°C abgekühlt. Methyliodid (6,2 ml in 20 ml THF) wurde zugegeben. Eine Lösung von Kalium-tert.-butylat (11,2 g) in THF (50 ml) wurde dann langsam zudosiert. Der Reaktionsverlauf bei -25°C wurde gaschromatographisch verfolgt. Nach 4h wurde auf RT erwärmt und die Reaktionsmischung mit 6%iger Ammoniaklösung gequencht. Nach Zusatz von Methyl-tert.-butylether wurden die Phasen getrennt und die organische Phase eingedampft. Der Rückstand wurde in Methyl-tert.-butylether aufgenommen und mit 1M HCl gewaschen. Die klare organische Phase wurde dann im Vakuum aufkonzentriert. Ausbeute 16,7 g Rohprodukt. Im ¹H-NMR waren neben der Zielverbindung noch mehrere, nicht identifizierbare Zersetzungprodukte zu erkennen. GC-Gehalt Rohprodukt: 79.0 Area%.

### Beispiel 9

***(2R,4R)-2-Tert.-butyl-3-formyl-1,3-thiazolidin-4-methyl-4-carbonsäuremethylester:*** In einem Laborreaktor wurde (2R,4R)-2-Tert.-butyl-3-formyl-1,3-thiazolidin-4-carbonsäuremethylester (20,8 g) vorgelegt, in wasserfreiem THF (130 ml) gelöst und auf -25°C abgekühlt. Methyliodid (6,2 ml in 20 ml THF) wurde zugegeben. Eine Lösung von Natrium-hexamethyldisilazid (49,8 ml einer 40%igen Lösung in THF) verdünnt in THF (50 ml) wurde dann langsam zudosiert. Der Reaktionsverlauf bei -25°C wurde gaschromatographisch verfolgt. Nach 3h wurde auf RT erwärmt und die Reaktionsmischung mit 15% Essigsäure (140 ml) gequencht. Die Phasen wurden getrennt, die organische Phase einmal mit Ammoniaklösung (12,5%; 120 ml) gewaschen und im Vakuum aufkonzentriert. Ausbeute 20,3 g Rohprodukt.
GC-Analyse Rohprodukt: 85.7 Area% (2R,4R)-2-Tert.-butyl-3-formyl-1,3-thiazolidin-4-methyl-4-carbonsäuremethylester, 10.7% N-Formyl-N-(1-methylthio-2,2-dimethyl-1-propyl)-dehydroalaninmethylester, restliche Peaks jeweils < 1 Area%.

### Beispiel 10

***(2R,4R)-2-Tert.-butyl-3-formyl-1,3-thiazolidin-4-methyl-4-carbonsäuremethylester:*** In einem Laborreaktor wurde (2R,4R)-2-Tert.-butyl-3-formyl-1,3-thiazolidin-4-carbonsäuremethylester (20,5 g) vorgelegt, in wasserfreiem THF (100 ml) gelöst und auf -25°C abgekühlt. Trifluormethansulfonsäuremethylester (11,0 ml) wurde zugegeben. Die Lithiumhexamethyldisilazidlösung (100 ml, 1M in THF) wurde dann langsam zudosiert. Die Reaktionsmischung wurde mit 15%iger Essigsäure gequencht, mit ges. NaCl-Lsg. verdünnt, die Phasen getrennt und die organische Phase eingedampft, in MTBE aufgenommen, mit H₂O gewaschen und im Vakuum aufkonzentriert. Ausbeute 21,8 g gelbes Öl (99%, enthielt noch Reste Essigsäure) (2R,4R)-2-Tert.-butyl-3-formyl-1,3-thiazolidin-4-methyl-4-carbonsäuremethylester. HPLC-Gehalt Rohprodukt: >95%. ¹H-NMR identisch mit dem Produkt aus Beispiel 5.

### Beispiel 11

***(2R,4R)-2-Tert.-butyl-3-formyl-1,3-thiazolidin-4-methyl-4-carbonsäuremethylester:*** In einem Kolben wurde (2R,4R)-2-Tert.butyl-3-formyl-1,3-thiazolidin-4-carbonsäuremethylester (9,95 g) vorgelegt, in wasserfreiem THF (60 ml) gelöst und im Wasser/Eisbad auf +5°C abgekühlt. Methyliodid (3,15 ml) in THF (9 ml) wurde zugegeben. Die Lithiumhexamethyldisilazidlösung (hergestellt aus 31,2 ml 1.6M Butyllithium in Hexan und Hexamethylsilazan (11,1 ml)) wurde dann langsam zudosiert, sodass die Temperatur unter 25°C verblieb. Die Reaktionsmischung wurde mit 15%iger Essigsäure gequencht und mit EtOAc verdünnt. Die Phasen wurden getrennt, die organische Phase einmal mit Ammoniaklösung (12,5%; 100 ml) gewaschen und im Vakuum aufkonzentriert. Ausbeute 10,8 g orange-braunes Öl (enthielt noch Reste Essigsäure und Silylverbindungen). ¹H-NMR entspricht dem von Beispiel 5. Gehalt an Reinverbindung betrug nach HPLC (kalibriert mit extrenem Standard) 69.9 Gew-%.

## Patentansprüche

1. Verfahren zur Herstellung von Verbindungen der allgemeinen Formel (2) 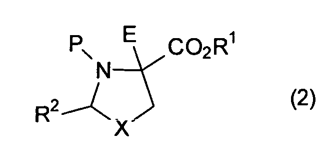 wobei
**X** für S oder O steht und
**R**^{**1**} ausgewählt wird aus der Gruppe enthaltend Wasserstoff, Metalle der ersten oder zweiten Hauptgruppe, lineare oder verzweigte C₁-C₁₂-Alkyl-, C₆-C₁₅-Aryl- oder C₇-C₂₁ Aralkyl-Reste, Dialkylsilyl- und Trialkylsilyl-, Dialkylarylsilyl-, Diarylalkylsilyl-, Triarylsilyl-Reste, wobei die organischen Reste an den Silylresten wiederum aus C₁-C₁₂-Alkyl und C₆-C₁₅-Aryl-Resten ausgewählt werden und
**R**^{**2**} ausgewählt wird aus der Gruppe enthaltend lineare oder verzweigte C₁-C₁₂-Alkyl-, C₆-C₁₅-Aryl- oder C₇-C₂₁ Aralkyl-Reste und
**P** für eine Aminoschutzgruppe steht und
**E** für einen Rest ausgewählt aus der Gruppe enthaltend gegebenenfalls durch Halogen-, Cyano-, Nitro- oder Ester-Gruppen substituierte, lineare oder verzweigte C₁-C₁₂-Alkyl-, C₃-C₁₀-Alkenyl-, C₆-C₁₅-Aryl- oder C₇-C₂₁-Aralkyl-Reste oder für eine Acyl-, oder Formylgruppe steht
durch Zugabe einer Base zu einer Reaktionsmischung enthaltend eine Verbindung der allgemeinen Formel (1) 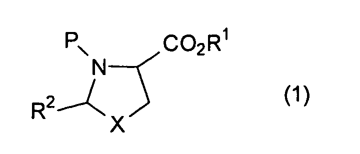 und ein Elektrophil E-Y
wobei
**Y** für eine Abgangsgruppe steht,
**dadurch gekennzeichnet, dass** die Reaktion bei einer Temperatur von größer - 40 °C durchgeführt wird.

2. Verfahren nach Anspruch 1, **dadurch gekennzeichnet, dass** zur Herstellung von optischen Isomeren in der Konfiguration der allgemeinen Formel (2a) 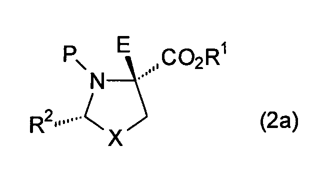 optische Isomere der allgemeinen Formeln (1a) oder (1b) in reiner Form oder als Gemische 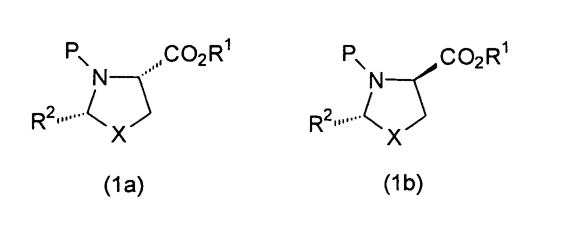 eingesetzt werden.

3. Verfahren nach Anspruch 1 bis 2, **dadurch gekennzeichnet, dass** P ausgewählt wird aus der Gruppe enthaltend Alkyl-, Formyl-, Acyl-, Oxycarbonyl-, Sulfonyl- , Sulfenyl- oder Silyl-Reste.

4. Verfahren nach Anspruch 1 bis 3, **dadurch gekennzeichnet, dass** Y ausgewählt wird aus der Gruppe enthaltend Halogene, Tosylate, Azide, Hydrazide, Dialkylamide oder Sulfonate.

5. Verfahren nach Anspruch 1 bis 4, **dadurch gekennzeichnet, dass** die Base ein Alkalimetallamid ist.

6. Verfahren nach Anspruch 1 bis 5, **dadurch gekennzeichnet, dass** E-Y für Methyliodid oder Dimethylsulfat steht.

7. Verfahren nach Anspruch 1 bis 6, **dadurch gekennzeichnet, dass** das Verfahren bei einer Temperatur von -30°C bis + 30°C durchgeführt wird.

8. Verfahren nach Anspruch 1 bis 7, **dadurch gekennzeichnet, dass** R¹ ausgewählt wird aus der Gruppe enthaltend Wasserstoff, Methyl, Ethyl, n-Propyl, Isopropyl, n-Butyl, tert-Butyl, Phenyl, Benzyl, Trimethylsilyl, Triethylsilyl oder Tributylsilyl.

9. Verfahren nach Anspruch 1 bis 8, **dadurch gekennzeichnet, dass** R² ausgewählt wird aus der Gruppe enthaltend Methyl, Ethyl, n-Propyl, Isopropyl, n-Butyl, tert-Butyl, Cyclohexyl, Phenyl, Tolyl, Naphthyl oder Benzyl.
